(19) Europäisches Patentamt
European Patent Office
Office européen des brevets

(11) Veröffentlichungsnummer: **0 665 213 A1**

# (12) EUROPÄISCHE PATENTANMELDUNG

(21) Anmeldenummer: **95100812.7**

(22) Anmeldetag: **21.01.95**

(51) Int. Cl.[6]: **C07C 231/12**, C07C 233/36, C07C 227/14, C07C 229/12

(30) Priorität: **29.01.94 DE 4402693**
**09.03.94 DE 4407840**

(43) Veröffentlichungstag der Anmeldung:
**02.08.95 Patentblatt 95/31**

(84) Benannte Vertragsstaaten:
**AT BE CH DE DK ES FR GB IT LI NL SE**

(71) Anmelder: **HOECHST AKTIENGESELLSCHAFT**

**D-65926 Frankfurt (DE)**

(72) Erfinder: **Vybiral, Reinhard, Dr.**
**Rossfeldstrasse 5**
**D-84508 Burgkirchen (DE)**
Erfinder: **Seitz, Hubert, Dr.**
**Thalhauserstrasse 27**
**D-84508 Burgkirchen (DE)**
Erfinder: **Aigner, Rudolf, DI (FH)**
**Hochkalterstrasse 7**
**D-84508 Burgkirchen (DE)**

## (54) Verfahren zur Herstellung von Betainen.

(57) Beim beschriebenen Verfahren zur Herstellung von Betainen wird das tertiäre Ausgangsamin zunächst mit einer ω-Halogenalkylcarbonsäure oder einem Salz davon vorzugsweise in Wasser als Lösungsmittel bei einer Temperatur von 60 bis 98 °C und einem pH-Wert von 7 bis 11 quaternisiert, wobei die tertiäre Aminverbindung und die Halogenalkylcarbonsäure oder deren Salz im Molverhältnis von 1 : 1 bis 1,5 eingesetzt worden sind. Die erhaltene Betainlösung wird dann bei einem pH-Wert von 1 bis 14 und einer Temperatur von 95 bis 170 °C so lange gehalten, bis kein organisch gebundenes Halogen mehr nachweisbar ist. Die fertige Betainlösung weist die geforderte Reinheit bezüglich Ausgangsamin und organischer Halogenverbindung auf.

EP 0 665 213 A1

Die Erfindung betrifft ein Verfahren zur Herstellung von Betainen der allgemeinen Formel 1

$$\begin{array}{c} R^2 \\ | \\ R^1\text{-}N^+\text{-}(CH_2)_y\text{-}COO^- \\ | \\ R^3 \end{array} \qquad (1)$$

worin $R^1$ ein Alkylrest mit mindestens 8 C-Atomen oder der Rest $R^4CONH(CH_2)_x$- ist, worin $R^4CO$ ein von einer Carbonsäure mit 6 bis 18 C-Atomen abgeleiteter Acylrest und x 2, 3 oder 4 ist, $R^2$ und $R^3$, gleich oder verschieden, einen Alkylrest mit 1 bis 4 C-Atomen oder den Rest $-(CH_2)_zOH$ mit z = 1, 2 oder 3 bedeuten und y 1, 2 oder 3 ist,
durch Quaternisierung von einem tertiären Amin der allgemeinen Formel 2

$$\begin{array}{c} R^2 \\ | \\ R^1\text{-}N \\ | \\ R^3 \end{array} \qquad (2)$$

worin $R^1$, $R^2$ und $R^3$ die angegebenen Bedeutungen haben, mit einer ω-Halogenalkylcarbonsäure der allgemeinen Formel 3

$X\text{-}(CH_2)_y\text{-}COOH$ (3)

worin X ein Halogen ist und y die genannte Bedeutung hat,
oder derem Salz in flüssiger Phase.

Ein solches Verfahren ist in der DE-A 42 05 880 beschrieben. Dabei wird die Quaternisierung in wäßriger Phase bei einer Temperatur von 115 bis 180 °C durchgeführt. Mit dieser relativ hohen Umsetzungstemperatur, die zu einem Abbau der eingesetzten ω-Halogenalkylcarbonsäure und der darin als Verunreinigung enthaltenen ω-Dihalogenalkylcarbonsäure führt, wird erreicht, daß die erhaltenen Betainlösungen nahezu frei sind von Verbindungen mit organisch gebundenem Halogen (Chlor) wie Natriummono- und -dichloracetat. Der Abbau von Halogenalkylcarbonsäure, das ist eine der beiden Reaktionskomponenten, führt aber auch dazu, daß die erhaltene Betainlösung eine mehr oder weniger große Menge an nicht umgesetztem Ausgangsamin enthält. Dem Vorteil, von organisch gebundenem Halogen freie Betainlösungen zu erhalten, steht also der Nachteil gegenüber, daß diese Lösungen mit der eingesetzten Aminverbindung verunreinigt sind.

In US-A 4 497 825 wird zur Erreichung von Betainlösungen, die frei sind von Ausgangsamin und organisch gebundenem Halogen, empfohlen, die Quaternisierungsreaktion bei einem pH-Wert von 7,5 bis 10,5 durchzuführen. Diese Betainlösungen enthalten zwar praktisch keine Aminverbindung mehr, wohl aber eine unerwünscht hohe Restmenge an organisch gebundenem Halogen in Form der eingesetzten Monohalogencarbonsäure und/oder deren Verunreinigung, nämlich Dihalogencarbonsäure, worauf auch in der obengenannten DE-A 42 05 880 hingewiesen wird.

Die Aufgabe der Erfindung besteht demnach darin, ein Verfahren zur Herstellung von Betainlösungen zur Verfügung zu stellen, die sowohl bezüglich organisch gebundenem Halogen als auch bezüglich Aminverbindungen die erwünschte Reinheit aufweisen, das heißt, der Gehalt an tertiärem Ausgangsamin soll < 0,5 Gew.-% und an Halogencarbonsäureverbindungen jeweils < 10 ppm sein.

Das erfindungsgemäße Verfahren ist dadurch gekennzeichnet, daß man

a) in einer ersten Reaktionsstufe, in der das tertiäre Amin und die ω-Halogenalkylcarbonsäure oder deren Salz in einem Molverhältnis von 1 : 1 bis 1,5, vorzugsweise 1 : 1,03 bis 1,3, eingesetzt werden, bei einer Temperatur von 60 bis 98 °C, vorzugsweise 70 bis 95 °C, und einem pH-Wert von 7 bis 11, vorzugsweise 8 bis 10, quaternisiert, wobei eine Betainlösung mit der gewünschten Reinheit bezüglich Ausgangsamin erhalten wird, und

b) in einer zweiten Reaktionsstufe die in der ersten Stufe erhaltene Betainlösung auf einen pH-Wert von 1 bis 14, vorzugsweise 5 bis 12, einstellt und bei einer Temperatur von 95 bis 170 °C, vorzugsweise 100 bis 150 °C, so lange hält, bis die Betainlösung auch die gewünschte Reinheit bezüglich organisch gebundenem Halogen aufweist.

Beim erfindungsgemäßen Verfahren wird die Umsetzung zwischen dem tertiären Amin und der Halogenalkylcarbonsäure oder dem Halogenalkylcarbonsäuresalz, das vorzugsweise ein Alkalimetallsalz ist (der Einfachheit halber wird von Halogenalkylcarbonsäure oder Halogencarbonsäure allein gesprochen), in der Weise durchgeführt, daß zunächst eine Betainlösung hergestellt wird, die weniger als 0,5 Gew.-% Ausgangsamin enthält, Gewichtsprozente bezogen auf die Lösung. Dies wird durch eine Kombination ausgewählter Werte bezüglich Molverhältnis der Reaktionskomponenten, Reaktionstemperatur und pH-Wert der Reaktionslösung während der Quaternisierung erreicht. So werden 1 bis 1,5 mol Halogenalkylcarbonsäure, vorzugsweise 1,03 bis 1,3 mol, pro mol tertiäre Aminverbindung eingesetzt. Der pH-Wert der Ausgangsmischung wird auf 7 bis 11, vorzugsweise 8 bis 10, eingestellt und bis zum Ende der Quaternisierung aufrechterhalten. Die Temperatur, bei der die Quaternisierungsreaktion vorgenommen wird, beträgt 60 bis 98 °C, vorzugsweise 70 bis 95 °C. Das Einstellen und Aufrechterhalten des genannten pH-Wertes der Mischung wird man (sofern dieser Wert nicht ohnehin vorliegt, wie zum Beispiel im Falle des Einsatzes von Halogenalkylcarbonsäuresalzen) durch Zugabe von einer vorzugsweise wäßrigen Alkalimetallhydroxidlösung vor und/oder während der Quaternisierungsreaktion durchführen. Die so erhaltene Betainlösung (die Reaktionszeit für die Quaternisierung beträgt etwa 6 bis 20 Stunden) ist rein bezüglich Ausgangsamin, nicht aber bezüglich eingesetzter Monohalogencarbonsäure und gegebenenfalls Dihalogencarbonsäure.

Diese Betainlösung wird nun in einer zweiten Stufe auf eine Temperatur von 95 bis 170 °C, vorzugsweise 100 bis 150 °C, gebracht und bei dieser Temperatur gehalten, bis die in Rede stehenden Halogencarbonsäureverbindungen abgebaut sind, bis also praktisch kein organisch gebundenes Halogen mehr anwesend ist. In der Betainlösung aus der ersten Stufe wird ferner ein pH-Wert von 1 bis 14, vorzugsweise 5 bis 12, eingestellt und aufrechterhalten, vorzugsweise mit Hilfe einer wäßrigen Mineral- oder Carbonsäure oder einer wäßrigen Alkalimetallhydroxidlösung. Die Zugabe der Säure- oder Hydroxidlösung zur Betainlösung aus der ersten Stufe kann vor und/oder während des Erhitzens auf die genannte Temperatur von 95 bis 170 °C, vorzugsweise 100 bis 150 °C, erfolgen. Bei dieser Temperatur und dem genannten pH-Wert von 1 bis 14, vorzugsweise 5 bis 12, wird die Betainlösung so lange gehalten, bis der Gehalt an organisch gebundenem Halogen < 10 ppm ist (die Reaktionszeit für den Abbau organisch gebundenen Halogens beträgt je nach Temperatur und pH-Wert im allgemeinen 3 bis 50 Stunden). Die so erhaltene Betainlösung weist nun die geforderte Reinheit bezüglich Amin und Halogencarbonsäureverbindung auf. Ihr Betaingehalt (Wirkstoffgehalt) beträgt im allgemeinen 20 bis 55 Gew.-%, vorzugsweise 25 bis 50 Gew.-%, das heißt, daß das Lösungsmittel (die flüssige Phase) in einer solchen Menge eingesetzt wird, daß Betainlösungen mit diesem Wirkstoffgehalt erhalten werden. Das Lösungsmittel kann Wasser, niedrige Alkohole, wie Methanol, Ethanol, Propanol, Isopropanol und/oder Propylenglykol, oder eine Mischung aus Wasser und Alkohol sein, wobei Wasser und Mischungen aus Wasser und Alkohol bevorzugt sind. Das Wasser und ebenso die anderen Lösungsmittel können als solche oder in Form von Lösungen von Alkalimetallhydroxid, Aminverbindung und/oder Halogencarbonsäureverbindung eingesetzt werden. Das erfindungsgemäße Verfahren kann diskontinuierlich oder kontinuierlich durchgeführt werden, zum Beispiel in einem oder in mehreren nebeneinander oder kaskadenförmig angeordneten Rührkesseln. Die erhaltenen Betainlösungen stellen bereits als solche wertvolle Produkte dar. Betaine sind nämlich vielfach einsetzbare oberflächenaktive Verbindungen. Aufgrund ihrer guten Hautverträglichkeit werden sie vor allem in der Körperpflege eingesetzt.

Bezüglich der Ausgangsverbindungen, tertiäres Amin, $\omega$-Monohalogencarbonsäure oder ein Salz davon, vorzugsweise ein Alkalimetallsalz, und gegebenenfalls Alkalimetallhydroxid, sei noch folgendes gesagt: Die tertiären Ausgangsamine entsprechen der eingangs angegebenen Formel 2. Der lange Alkylrest $R^1$ kann auch Doppelbindungen enthalten, vorzugsweise 1 bis 3. Bevorzugte Ausgangsamine sind solche der Formel 2, wenn $R^1$ ein Alkylrest mit 8 bis 18 C-Atomen ist oder ein Rest der Formel $R^4CONH(CH_2)_x$-, worin $R^4CO$ ein von einer Carbonsäure mit 6 bis 18 C-Atomen abgeleiteter Acylrest und x 2, 3 oder 4 ist, und $R^2$ und $R^3$ jeweils Methyl sind. Als Beispiele seien genannt: Dimethyloctylamin, Dimethyllaurylamin, Dimethylstearylamin, Dimethylcocosalkylamin, Dimethyltalgalkylamin und dergleichen sowie Lauroylaminopropyldimethylamin, Stearoylaminopropyldimethylamin, Cocosacylaminopropyldimethylamin und dergleichen. Die $\omega$-Halogencarbonsäure ist vorzugsweise die Monochloressigsäure beziehungsweise Monochlornatriumacetat. Das Alkalimetallhydroxid ist vorzugsweise Natriumhydroxid oder Kaliumhydroxid.

Die Erfindung wird nun an erfindungsgemäßen Beispielen und Vergleichsbeispielen noch näher erläutert.

Erfindungsgemäße Beispiele

Beispiele 1 bis 3 zur ersten Stufe des erfindungsgemäßen Verfahrens:

Beispiel 1

In einem 1-l-Glaskolben, ausgerüstet mit Rührer, Thermometer, Rückflußkühler und Tropftrichter, werden 188 g (0,587 mol) Cocosamidopropyl-N,N-dimethylamin (Amidamin) und 345 g Wasser vorgelegt. Die Mischung wird unter Rühren auf etwa 82 °C erwärmt. Zu dieser Suspension werden unter Beibehaltung der genannten Temperatur von etwa 82 °C langsam und kontinuierlich in 5,5 Stunden 72,8 g (0,616 mol) einer 80gew.%igen wäßrigen Monochloressigsäurelösung zugetropft sowie 53,7 g (0,671 mol) einer 50gew.%igen wäßrigen NaOH-Lösung zur Einstellung eines pH-Wertes von 8 bis 9 (das Molverhältnis von Amidamin zu Monochloressigsäure ist 1 : 1,05). Nach beendeter Zugabe wird 9 Stunden lang bei etwa 80 °C nachreagieren gelassen. Die erhaltene 30gew.%ige wäßrige Betainlösung hat bezüglich Cocosamidamin, Monochloressigsäure (MCE) und Dichloressigsäure (DCE) die folgenden Gewichtsprozentwerte beziehungsweise ppm-Werte, bezogen auf die Lösung:

| | |
|---|---|
| Amidamin: | 0,14 % |
| MCE: | 0,1 % |
| DCE: | 110 ppm |

Beispiel 2

Beispiel 1 wird wiederholt unter Anwendung der folgenden Änderungen:

| | |
|---|---|
| Molverhältnis von Amidamin: | MCE = 1 : 1,25 |
| Temperatur: | 95 °C |
| pH-Wert: | 8 bis 9 |
| Nachreaktionszeit: | 7 Stunden |

| Ergebnis: | |
|---|---|
| Amidamin: | 0,11 % |
| MCE: | 0,13 % |
| DCE: | 100 ppm |

Beispiel 3

Beispiel 1 wird wiederholt unter Anwendung der folgenden Änderungen:

| | |
|---|---|
| Molverhältnis von Amidamin: | MCE = 1 : 1,05 |
| Temperatur: | 70 °C |
| pH-Wert: | 8 bis 9 |
| Nachreaktionszeit: | 12 Stunden |

| Ergebnis: | |
|---|---|
| Amidamin:<br>MCE:<br>DCE: | 0,25 %<br>0,09 %<br>115 ppm |

Die Betainlösungen haben also bezüglich Amidamin den gewünschten niedrigen Wert, nicht jedoch bezüglich MCE und DCE.

Beispiele 4 bis 17 zur zweiten Stufe des erfindungsgemäßen Verfahren:
In der zweiten Stufe wird der MCE- und DCE-Gehalt der Betainlösung der ersten Stufe bis in den ppm-Bereich reduziert unter Beibehaltung der niedrigen Amidaminwerte. Aus Zweckmäßigkeitsgründen wird nur die Betainlösung des Beispiels 1 eingesetzt.

Beispiel 4

Die Betainlösung von Beispiel 1 wird mit 50gew.%iger wäßriger Natronlauge auf einen pH-Wert von 12 eingestellt und anschließend in einem Rührautoklaven bei einer Temperatur von 105 °C 48 Stunden lang gerührt. Die erhaltene Betainlösung weist nun einen MCE- und DCE-Gehalt von jeweils < 10 ppm auf.

Beispiele 5 bis 18

Die Betainlösung von Beispiel 1 wird mit 50gew.%iger wäßriger Natronlauge (Beispiele 5 bis 15) oder mit 30 bis 36gew.%iger wäßriger Salzsäure (Beispiele 16 bis 18) auf einen bestimmten pH-Wert eingestellt und anschließend in einem Rührautoklaven bei einer bestimmten Temperatur mehr oder weniger lang (Reaktionszeit) gerührt. Diese Reaktionsbedingungen und das Ergebnis bezüglich MCE- und DCE-Gehalt sind in der nachstehenden Tabelle gemeinsam mit den Werten des Beispiels 4 zusammengefaßt.

Mit dem erfindungsgemäßen Verfahren werden also Betainlösungen erhalten, die sowohl bezüglich Ausgangsamin als auch bezüglich halogenierten organischen Verbindungen (MCE und DCE) den jeweils geforderten niedrigen Gehalt aufweisen.

**Tabelle**

| Beispiel-Nr. | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|
| pH-Wert | 12 | 12 | 12 | 14 | 14 | 8 | 10 | 12 | 14 | 14 | 14 | 14 | 6 | 4,5 | 2 |
| Temperatur (°C) | 105 | 110 | 115 | 115 | 120 | 125 | 125 | 135 | 95 | 110 | 105 | 115 | 135 | 135 | 135 |
| Reaktionszeit (h) | 48 | 20 | 20 | 16 | 3 | 24 | 16 | 3 | 50 | 16 | 20 | 6 | 5 | 4 | 5 |
| MCE-Gehalt (ppm) | < 10 | < 10 | < 10 | < 10 | < 10 | < 10 | < 10 | < 10 | < 10 | < 10 | < 10 | < 10 | < 10 | < 10 | < 10 |
| DCE-Gehalt (ppm) | < 10 | < 10 | < 10 | < 10 | < 10 | < 10 | < 10 | < 10 | < 10 | < 10 | < 10 | < 10 | < 10 | < 10 | < 10 |

Vergleichsbeispiele (Nacharbeitung der Beispiele 2.1 und 2.3 von DE-A 42 05 880)

Vergleichsbeispiel 1

In einem 1-l-Rührautoklaven werden 59,3 g (0,51 mol) Natriummonochloracetat, 154,3 g (0,5 mol) Cocosamidopropyl-N,N-dimethylamin (Cocosamidamin) und 355,7 g Wasser vorgelegt und auf 120 °C erhitzt, wobei sich ein Druck von 2,6 bar einstellt. Nach einer Reaktionszeit von 8 Stunden wird die Reaktionslösung abgekühlt. Das Produkt ist durch die folgenden Daten charakterisiert:

| | |
|---|---|
| Natriumchloridgehalt: | 5,2 % |
| Cocosamidamingehalt: | 2,4 % |
| Glykolsäuregehalt: | 0,37 % |
| Natriummonochloracetatgehalt: | < 20 ppm |
| Natriumdichloracetatgehalt: | < 10 ppm |

Vergleichsbeispiel 2

Vergleichsbeispiel 1 wird bei einer Reaktionstemperatur von 140 °C wiederholt. Es stellt sich ein Druck von 3,2 bar ein. Die Reaktionszeit beträgt wieder 8 Stunden. Das Produkt ist durch die folgenden Daten charakterisiert:

| | |
|---|---|
| Natriumchloridgehalt: | 5,2 % |
| Cocosamidamingehalt: | 2,5 % |
| Glykolsäuregehalt: | 0,42 % |
| Natriummonochloracetatgehalt: | < 20 ppm |
| Natriumdichloracetatgehalt: | < 10 ppm |

**Patentansprüche**

**1.** Verfahren zur Herstellung von Betainen der allgemeinen Formel 1

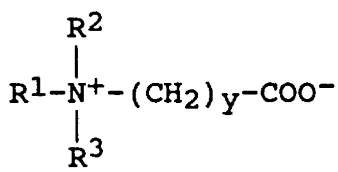

(1)

worin $R^1$ ein Alkylrest mit mindestens 8 C-Atomen oder der Rest $R^4CONH(CH_2)_x$- ist, worin $R^4CO$ ein von einer Carbonsäure mit 6 bis 18 C-Atomen abgeleiteter Acylrest und x 2, 3 oder 4 ist, $R^2$ und $R^3$, gleich oder verschieden, einen Alkylrest mit 1 bis 4 C-Atomen oder den Rest $-(CH_2)_zOH$ mit z = 1, 2 oder 3 bedeuten und y 1, 2 oder 3 ist,
durch Quaternisierung von einem tertiären Amin der allgemeinen Formel 2

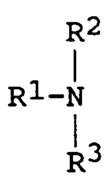

(2)

worin $R^1$, $R^2$ und $R^3$ die angegebenen Bedeutungen haben,

mit einer ω-Halogenalkylcarbonsäure der allgemeinen Formel 3

$X\text{-}(CH_2)_y\text{-}COOH$ (3)

worin X ein Halogen ist und y die genannte Bedeutung hat,
oder derem Salz in flüssiger Phase, dadurch gekennzeichnet, daß man

a) in einer ersten Reaktionsstufe, in der das tertiäre Amin und die ω-Halogenalkylcarbonsäure oder deren Salz in einem Molverhältnis von 1 : 1 bis 1,5 eingesetzt werden, bei einer Temperatur von 60 bis 98 °C und einem pH-Wert von 7 bis 11 quaternisiert, wobei eine Betainlösung mit der gewünschten Reinheit bezüglich Ausgangsamin erhalten wird, und

b) in einer zweiten Reaktionsstufe die in der ersten Stufe erhaltene Betainlösung auf einen pH-Wert von 1 bis 14 einstellt und bei einer Temperatur von 95 bis 170 °C so lange hält, bis die Betainlösung auch die gewünschte Reinheit bezüglich organisch gebundenem Halogen aufweist.

**2.** Verfahren nach Anspruch 1, dadurch gekennzeichnet, daß man das tertiäre Amin und die ω-Halogenalkylcarbonsäure oder deren Salz in einem Molverhältnis von 1 : 1,03 bis 1,3 einsetzt und bei einer Temperatur von 70 bis 95 °C und einem pH-Wert von 8 bis 10 quaternisiert.

**3.** Verfahren nach Anspruch 1 oder 2, dadurch gekennzeichnet, daß man die Reaktionsstufe b) bei einer Temperatur von 100 bis 150 °C und einem pH-Wert von 5 bis 12 durchführt.

**4.** Verfahren nach einem oder mehreren der Ansprüche 1 bis 3, dadurch gekennzeichnet, daß man die Reaktionsstufen a) und b) in Wasser als flüssiger Phase durchführt.

**5.** Verfahren nach Anspruch 4, dadurch gekennzeichnet, daß man das Wasser in einer solchen Menge einsetzt, daß die fertige Betainlösung einen Betaingehalt von 20 bis 55 Gew.-% aufweist, Gewichtsprozente bezogen auf die Lösung.

Europäisches Patentamt

# EUROPÄISCHER RECHERCHENBERICHT

Nummer der Anmeldung
EP 95 10 0812

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X<br><br><br>D | EP-A-0 557 835 (TH. GOLDSCHMIDT AG.)<br>* Seite 6, Zeile 43 - Zeile 56; Ansprüche 1-3 *<br>& DE-A-42 05 880<br>--- | 1-5 | C07C231/12<br>C07C233/36<br>C07C227/14<br>C07C229/12 |
| X<br><br><br>D | EP-A-0 020 907 (TH. GOLDSCHMIDT AG)<br>* Seite 4, Zeile 10 - Zeile 28; Ansprüche 1,2; Beispiele 1,3 *<br>& US-A-4 497 825<br>--- | 1-5 | |
| A | EP-A-0 563 747 (HOECHST AG)<br>* Seite 4, Zeile 8 - Zeile 12; Ansprüche 1-6 *<br>----- | 1-5 | |
| | | | RECHERCHIERTE SACHGEBIETE (Int.Cl.6) |
| | | | C07C |

Der vorliegende Recherchenbericht wurde für alle Patentansprüche erstellt

| Recherchenort | Abschlußdatum der Recherche | Prüfer |
|---|---|---|
| BERLIN | 11.April 1995 | Rufet, J |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenharung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P04C03)